(19) Europäisches Patentamt
European Patent Office
Office européen des brevets

(11) EP 0 736 521 A1

(12) **DEMANDE DE BREVET EUROPEEN**

(43) Date de publication:
**09.10.1996 Bulletin 1996/41**

(21) Numéro de dépôt: **96400728.0**

(22) Date de dépôt: **04.04.1996**

(51) Int Cl.6: **C07C 227/40**, C07C 231/24, C07C 303/44, C07C 229/12, C07C 233/36, C07C 309/14, B01D 61/44, C11D 1/88, C11D 1/90, C11D 1/92, C11D 3/00

(84) Etats contractants désignés:
**DE FR GB IT**

(30) Priorité: **05.04.1995 FR 9504014**

(71) Demandeur: **RHONE-POULENC CHIMIE**
**92408 Courbevoie Cédex (FR)**

(72) Inventeur: **Derian, Paul-Joel**
**Lawrenceville, NJ 08648 (US)**

(74) Mandataire: **Dubruc, Philippe**
**RHONE-POULENC CHIMIE,**
**Direction de la Propriété Industrielle,**
**25, Quai Paul Doumer**
**92408 Courbevoie Cédex (FR)**

(54) **Procédé de purification de tensio-actifs amphotères par électrodialyse**

(57) La présente invention concerne un procédé de purification de tensio-actifs de type amphotère ou bétaïne selon lequel on soumet une solution aqueuse dudit tensio-actif à une électrodialyse, caractérisé en ce que ladite solution contient de 0,1 à 10%, de préférence de 0,5 à 5% en poids d'un alcool, plus particulièrement d'un polyol par rapport au poids du tensio-actif.

Utilisation des tensio-actifs purifiés notamment dans les compositions détergentes ou cosmétiques.

EP 0 736 521 A1

## Description

La présente invention concerne un procédé de purification de tensio-actifs de type amphotère par électrodialyse.

Il existe déjà de nombreux tensio-actifs de type amphotère ou bétaïne (c'est-à-dire présentant une charge électrique globale de zéro) qui sont utilisés comme agents détergents et/ou dispersants et/ou émulsifiants dans diverses compositions cosmétiques et détergentes.

Cependant depuis quelques années, il existe un besoin très important de tensio-actifs du type ci-dessus qui soient à la fois efficaces et qui ne soient pas irritants pour la peau ou les yeux et qui soient utilisables sans précautions particulières avec le maximum de sécurité. Les recherches ont convergé sur des tensio-actifs amphotères du type bétaïne ou amphoacétate dont les procédés de synthèse sont généralement basés sur une réaction de carboxylation d'une amine grasse, par exemple une alkylamidoéthyléthanolamine ou une alkylimidazoline, sur l'acide monochloroacétique ou son sel de sodium. Cette synthèse conduit donc à la libération de $Na^+ Cl^-$, l'anion $Cl^-$ provenant de l'acide monochloroacétique, ainsi qu'à la libération de glycolate de sodium provenant de l'hydrolyse de l'acide de départ.

Les inconvénients majeurs de la présence de NaCl dans les tensioactifs du type ci-dessus sont les suivants :

- augmentation importante de la viscosité de la solution de tensio-actif qui ne permet pas de concentrer les solutions au-delà d'une concentration en poids de 30% environ car la viscosité augmente brusquement, ce qui empêche la commercialisation de solutions concentrées,
- détérioration de la tenue à froid et des propriétés émulsifiantes et stabilisantes,
- problème de compatibilité avec les autres additifs des compositions (agents épaississants, conservateurs, émulsions de silicones, etc.).

Il n'existe pas de procédé industriel de synthèse permettant d'aboutir à un produit final exempt de produit ionique.

Parmi les divers procédés possibles de purification du produit final, le procédé d'électrodialyse de solutions aqueuses de sels alcalins de tensio-actifs du type bétaïne, par exemple celui enseigné par EP-A 269.940, permettrait d'obtenir moins de 0,2 mole de sel minéral pour 100 g de tensio-actif.

Selon ce procédé la solution est placée dans une série de cellules limitées par une membrane anionique et une membrane cationique et on fait passer un courant électrique à travers ce système.

Ce procédé est certes efficace mais il présente un problème majeur consistant en un colmatage rapide des membranes anioniques qui a pour conséquence une diminution de la densité de courant et de rendement faradique dans les cellules.

Ce problème a été résolu par la présente invention qui concerne en effet un procédé de purification de tensio-actif de type amphotère ou bétaïne selon lequel on soumet une solution aqueuse dudit tensio-actif à une électrodialyse, caractérisé en ce que ladite solution contient de 0,1 à 20%, de préférence de 0,5 à 10 % en poids d'un alcool par rapport au poids du tensio-actif.

Par tensio-actif de type bétaïne on entend selon l'invention :

☐ les bétaïnes de formule 1 :

$$R_1\ R_2\ R_3\ \overset{+}{N}R_4\ C(O)O^- \qquad (1)$$

☐ les sulfo-bétaïnes de formule 2 :

$$R_1\ R_2\ R_3\ \overset{+}{N}R_4\ SO_3^- \qquad (2)$$

☐ les amidoalkylbétaïnes de formule 3 :

$$R_1C(O)\text{ - }NH\ R_2\overset{+}{N}\ (R_3\ R_4)\ R_5\ C(O)O^- \qquad (3)$$

☐ et les sulfo-bétaïnes de formule 4:

$$R_1C(O)\text{ - }NH\ R_2\overset{+}{N}\ (R_3\ R_4)\ R_5\ SO_3^- \qquad (4)$$

formules dans lesquelles les radicaux $R_1$ représentent un radical alkyle ou alcényle de 10 à 24 atomes de carbone, $R_2$, $R_3$, $R_4$, et $R_5$ identiques ou différents représentent un radical alkyle ou alkylène ayant de 1 à 4 atomes de carbone.

Outre ces tensio-actifs la présente invention vise plus particulièrement les tensio-actifs amphotères comme les amphoacétates, et plus particulièrement les dérivés d'amidoamines répertoriés dans le dictionnaire "Cosmetics Toiletries Fragrance Association" Edition 1994 sous les noms de cocoamphoacétate et répondant plus généralement à la formule (5) :

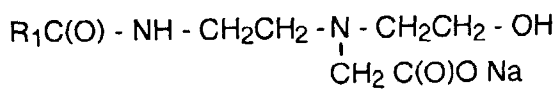
(5)

dans laquelle $R_1$ a la signification ci-dessus et représente le plus souvent des chaînes coco et lauryle.

Un procédé généralement utilisé pour préparer les amidoéthylbétaïnes est la réaction d'une alkylimidazoline de formule (6).

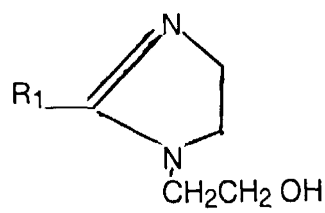
(6)

dans laquelle $R_1$ a la signification donnée ci-dessus ou d'un de ses dérivés à cycle ouvert avec un acide monohalogénoacétique, de préférence l'acide chloroacétique ou l'un de ses sels en présence d'un alcali (voir notamment US-A 2.528.378 et US-A 2.773.068).

De préférence le pH du mélange réactionnel est réglé pour qu'il ne dépasse pas 10 durant la réaction (voir demande de brevet européen 94.402.226 déposée le 5 octobre 1994).

Dans le cas particulier du Miranol, c'est-à-dire le produit de formule (7) dans laquelle $R_2 = R_3 = -CH_2-CH_2$, l'alcali est de préférence la soude. De plus le pH est maintenu de préférence entre 8,5 et 9,5. Par ailleurs, la température de la réaction est plus particulièrement comprise entre 75 et 85°C.

Selon une variante, l'imidazoline est préparée par réaction d'un triglycéride gras comme l'huile de coprah, sur l'aminoéthyléthanolamine. Dans le milieu réactionnel apparaît alors du glycérol comme produit de réaction à une teneur qui peut être suffisante pour le procédé d'électrodialyse car le glycérol se trouve directement dans la solution de tensio-actif à une teneur comprise entre 3 et 8 % en poids par rapport au poids de l'amphoacétate. Le tensio-actif est obtenu ensuite de façon classique par l'action d'acide chloromonoacétique.

Le procédé de purification selon l'invention est particulièrement efficace quand on traite l'alkylamphoacétate préparé selon le procédé ci-dessus. Il est aussi efficace lorsque l'on traite un alkylamphoacétate préparé par réaction d'une aminoalkylalcanolamine de formule (7) :

$$R_1\ C(O)\ NH\ R_2\ NH\ R_3 - OH \qquad (7)$$

dans laquelle $R_1$, $R_2$ et $R_3$ ont la signification indiquée ci-dessus avec du formaldéhyde et un cyanure de formule (8):

$$XCN \qquad (8)$$

dans laquelle X représente un atome d'hydrogène ou un métal alcalin, sous réserve que si X est un atome d'hydrogène, on hydrolyse le nitrure obtenu avec l'alcali.

Un tel procédé au cyanure est décrit dans la demande de brevet britannique nº 94.23573 déposée le 22 novembre

1994. L'alcali est de préférence la soude.

On utilise de préférence au moins une mole de cyanure et de formaldéhyde par mole de composé de formule (7), de préférence entre 1 et 1,2 mole, le produit réactionnel obtenu étant décoloré au peroxyde d'hydrogène.

L'alcool utilisé dans le cadre de la présente invention est de préférence un alcool comportant par molécule au moins 1 groupe hydroxy, de préférence au moins 2 groupe hydroxy, et dont la partie aliphatique hydrocarbonée est linéaire ou branchée et comporte de 2 à 20, de préférence de 2 à 10, atomes de carbone et peut être interrompue par un ou des hétéroatomes choisis parmi l'oxygène, le soufre et l'azote. Comme alcool on recommande d'utiliser un alcool qui soit liquide à la température à laquelle on effectue l'électrodialyse et qui est généralement comprise entre 20 et 80 °C. Comme polyol, on recommande d'utiliser le dipropylèneglycol, le propanediol 1-2, le butanediol 1-3 et le glycérol.

Selon une variante préférée, on maintient la solution de tensio-actif durant l'électrodialyse à un pH compris entre 4 et 10, de préférence entre 6 et 8, de préférence à pH 7.

Le pH est réglé en ajoutant soit une base, de préférence la soude, soit un acide, de préférence l'acide chlorhydrique.

Cette action sur le pH permet de diminuer encore la polarisation et le colmatage des membranes.

Les électrodialyseurs sont des dispositifs connus disponibles commercialement. Ils sont constitués d'une suite de cellules d'électrolyse généralement montées en parallèle.

Ces cellules comportent plusieurs compartiments d'électrolyse formés chacun de deux membranes, à savoir une membrane cationique perméable aux cations et imperméable aux anions et une membrane anionique perméable aux anions et imperméable aux cations.

Les compartiments de dilution où l'électrolyte est extrait, alternent avec les compartiments de concentration où l'électrolyte se concentre.

Chaque cellule comporte un ou plusieurs compartiments de dilution et de concentration. Les électrodialyseurs industriels comportent généralement deux circuits hydrauliques alimentant respectivement en parallèle l'ensemble des compartiments du même type. Les cations vont migrer vers la cathode et les anions vers l'anode en traversant respectivement les membranes anioniques et cationiques. Grâce à une disposition alternée des membranes, les ions capables de traverser les membranes cationiques sont arrêtés par les membranes anioniques et réciproquement.

L'électrodialyse permet donc de transférer des électrolytes du circuit de dilution vers le circuit de concentration. Un troisième circuit alimente les électrodes qui produisent le courant traversant en série l'ensemble des compartiments. On utilise une densité de courant généralement comprise entre 1 et 50, de préférence entre 5 et 30 $mA/cm^2$.

Comme membrane on peut utiliser les membranes vendues sous les marques "Selemion" ou "Neosepta" et qui satisfont aux normes nouvelles de l'électrodialyse. Il en est de même pour les électrodes qui sont généralement en Titane ou en Niobium platiné.

La tension appliquée aux bornes d'une cellule ne doit pas être trop élevée pour éviter la polarisation du système. Une tension appliquée aux bornes de chaque cellule, comprise entre 0,5 et 30 V convient généralement.

On cherche à effectuer l'électrodialyse à une température la plus élevée possible ce qui augmente la mobilité électrolytique et réduit la viscosité de la solution. La limite supérieure en température est fonction en fait de la résistance des membranes utilisées à la chaleur.

La présente invention a également pour objet les compositions cosmétiques à base des tensio-actifs de type amphotère ou bétaïne purifiés par le procédé de dialyse faisant l'objet de l'invention.

On entend par le terme composition ou formulation cosmétique tous les produits ou préparations cosmétiques comme celles décrites dans l'annexe I ("Illustrative list by category of cosmetic products") de la directive européenne n° 76/768/CEE du 27 juillet 1976 dite "directive cosmétique".

Les tensio-actifs de type amphotère ou bétaïne purifiés par le procédé faisant l'objet de la présente invention peuvent être utilisés plus particulièrement dans des formulations de lotions, laits de toilettes, compositions démaquillantes, crèmes de soins ou crèmes ou lotions de protection contre le soleil et le rayonnement ultraviolet, mousses, gels coiffants ou toute formulation utilisée pour le coiffage ou pour faciliter le peignage des cheveux, shampooings pour cheveux ou le corps, gels de nettoyage du visage ou du corps, savons liquides, compositions moussantes pour le bain, formulations utilisées pour le nettoyage des dents ou de la cavité buccale comme les bains de bouche ou les dentifrices. Lesdits tensio-actifs de type amphotère ou bétaïne peuvent aussi être utilisés seuls ou en association dans la formulation de savons ou de pains de toilette. De part leur activité cationique à pH acide, ils peuvent aussi être mis en oeuvre pour la confection de formulations de rinçage ou de baumes conditionneurs pour les cheveux ou la peau.

Les compositions cosmétiques contiennent généralement ces tensioactifs servant à disperser, émulsionner, solubiliser, stabiliser divers composés utilisés pour leurs propriétés émollientes ou humectantes. Les tensioactifs sont utilisés dans ces compositions à des concentrations variant de 0,05 à 10% en poids de la préparation. Parmi les agents humectants, on peut citer le glycérol, le sorbitol, l'urée, le collagène, la gélatine, l'aloe vera, l'acide hyaluronique, ...

Les émollients sont généralement choisis parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson,...), des dérivés de ces huiles comme les huiles hydrogénées, les dérivés de la lanoline, les huiles minérales

ou les huiles paraffiniques, le perhydrosqualane, le squalène, les diols comme le 1-2-propanediol, le 1-3-butanediol, l'alcool cétylique, l'alcool stéarylique, l'alcool oléique, les polyéthylèneglycols ou polypropylèneglycols, les esters gras comme le palmitate d'isopropyle, le cocoate d'éthyl-2-hexyle, le myristyl myristate, les esters de l'acide lactique, l'acide stéarique, l'acide béhennique, l'acide isostéarique, les huiles silicones regroupant les polydiméthylsiloxanes cycliques, les polydiméthylsiloxanes $\alpha$-$\omega$ hydroxylées, les polydiméthylsiloxanes $\alpha$-$\omega$ triméthylsilyllés, les polyorganosiloxanes comme les polyalkylméthylsiloxanes, les polyméthylphénylsiloxanes, les polydiphénylsiloxanes, les dérivés aminés des silicones, les cires silicones, les silicones copolyéthers (comme l'huile SILBIONE 70646® commercialisée par la société RHONE-POULENC ou DC 190® commercialisée par DOW CORNING) ou les dérivés mixtes de silicones comme les copolymères mixtes polyalkylméthylsiloxanes-silicones copolyéthers.

A ces composés, on peut ajouter des poudres ou des particules minérales comme du carbonate de calcium, des oxydes minéraux sous forme de poudre ou sous forme colloïdale (particules de taille inférieure ou de l'ordre de un micromètre, parfois de quelque dizaines de nanomètres) comme du dioxyde de titane, de la silice, des sels d'aluminium utilisés généralement comme antitranspirants, du kaolin, du talc, des argiles et leurs dérivés,...

Des agents conservateurs comme les méthyl, éthyl, propyl et butyl esters de l'acide p-hydroxybenzoïque, le benzoate de sodium, le GERMABEN® ou tout agent chimique évitant la prolifération bactérienne ou des moisissures et utilisé traditionnellement dans les compositions cosmétiques sont généralement introduits dans ces compositions à hauteur de 0,01 à 3% en poids.

Alternativement à ces agents chimiques, on peut parfois utiliser des agents modifiants l'activité de l'eau et augmentant fortement la pression osmotique comme les carbohydrates ou des sels.

Pour protéger la peau ou les cheveux des agressions du soleil et des rayons UV, on peut ajouter à ces formulations des filtres solaires qui sont, soit des composés chimiques absorbant fortement le rayonnement UV comme les composés autorisés dans la directive Européenne N° 76/768/CEE, ses annexes et les modifications ultérieures de cette directive, soit le dioxyde de titane ou les oxydes de cérium sous forme de poudre ou de particules colloïdales.

Des parfums, des colorants ou des pigments peuvent également être ajoutés. Peuvent aussi être présents des polymères viscosants ou gélifiants (comme les polyacrylates réticulés CARBOPOL® commercialisés par GOODRICH-, les dérivés de la cellulose comme l'hydroxypropylcellulose, la carboxyméthylcellulose, les guars et leurs dérivés, la caroube, la gomme de tara ou de cassia, la gomme xanthane, les alginates, les carraghénannes, les dérivés de la chitine comme le chitosan,...

Un dentifrice peut contenir, outre des agents abrasifs comme de la silice ou le carbonate de calcium précipité, le phosphate dicalcique, le carbonate de sodium (utilisés seuls ou en combinaison à une teneur de 5 à 60% en poids du total de la composition), de 0,1 à 5% en poids desdits tensioactifs de l'invention, pris seuls ou en mélange avec les tensio-actifs. Les compositions de dentifrices comportent aussi de 5 à 85% d'agents dits humectants comme le glycérol, le sorbitol, les polyéthylèneglycols, le lactilol, le xylitol.

Le comportement rhéologique de la pâte dentaire, c'est-à-dire sa viscosité, sa tenue sur la brosse, la facilité de son extrusion à la sortie du tube ou du dispenseur, le caractère non filant de la pâte est contrôlé par des agents épaississants comme certaines silice utilisées à cet effet (TIXOSIL 43® commercialisées par RHONE-POULENC) et/ou des polymères utilisés seuls ou en association comme la gomme Xanthane, la gomme guar, les dérivés de la cellulose (Carboxyméthylcellulose par exemple), des polyacrylates réticulés comme les CARBOPOL® distribués par GOODRICH, des alginates ou des carraghénannes, de la VISCARIN®. Le total des agents épaississants représente de 0.1 à 15% en poids de la composition d'un dentifrice.

On retrouve aussi généralement associés à ces divers constituants des agents thérapeutiques comme certains sels de fluor ou de potassium, des arômes, des agents édulcorants et de l'eau.

Les tensio-actifs purifiés de type amphotère ou bétaïne de cette invention peuvent être aussi utilisés dans des formulations de pains de toilettes appelées savonnettes ou savons.

Les compositions classiques de pains de toilette comprennent généralement des sels d'acide gras utilisés en association avec des tensioactifs de l'invention et éventuellement des tensio-actifs autres que les sels d'acides gras ou les acides gras eux-mêmes. Ces compositions peuvent même ne contenir aucun acide gras ou sel d'acide gras et leurs formulations reposent alors sur d'autres tensioactifs comme par exemple les alkyl iséthionates de sodium en $C_8$-$C_{22}$ ou les alkylsulfates de sodium en $C_8$-$C_{22}$.

A ces compositions on peut aussi ajouter différents constituants utiles pour diminuer l'irritation ou l'agression de la peau comme les sels de métaux alcalins ou les iséthionates ou pour favoriser l'hydratation de la peau comme certains carbohydrates (glycérol, sorbitol par exemple), des polyéthylènes glycol ou polypropylène glycol, des dérivés alcoxylés des sucres ou de leurs dérivés (méthyl glucose par exemple), des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation comme la gomme xanthane et les dérivés de ces polycarbohydrates comme les celluloses modifiées (par exemple hydroxyéthylcellulose, carboxyméthylcellulose, celluloses cationiques comme les POLYMER JR® commercialisés par la société UNION CARBIDE), les dérivés du guar ou de la caroube comme leurs dérivés cationiques

(JAGUAR C13S®, JAGUAR C162® commercialisés par RHONE-POULENC) ou des dérivés non-ioniques (par exemple hydroxypropylguar), les dérivés anioniques (carboxyméthylguar) ou les dérivés mixtes non-ioniques/anioniques comme les carboxy-hydroxypropyl-guars ou non-ioniques/cationiques. On peut aussi ajouter alternativement ou en association des polymères synthétiques comme les polyacrylates ou des polymères cationiques synthétiques, connus sous le nom générique CTFA de "Polyquaternium", par exemple les polymères MIRAPOL A15® ou MIRAPOL 550® de la société RHONE-POULENC. On peut aussi ajouter à ces compositions des dérivés alcoxylés de glucose comme le GLUCAM P20 commercialisé par AMERCHOL et utilisés comme agent de texture pour la mousse.

On peut aussi avantageusement ajouter à ces compositions des agents séquestrants des métaux, plus particulièrement ceux séquestrants du calcium comme les ions citrates ou des agents émollients comme les silicones ou des huiles ou corps gras utilisés à ce propos dans l'industrie cosmétique (huiles minérales, esters d'acides gras, triglycérides, silicones, ...).

A ces ingrédients on rajoute généralement un ou des parfums, des colorants et/ou des agents opacifiants comme des pigments (particules d'oxyde de titane). On peut aussi incorporer dans la composition des agents bactéricides ou fongicides afin d'améliorer la désinfection de la peau.

Dans un pain de toilette dont la formulation est constituée principalement de savons d'acides gras monocarboxyliques (sels de sodium, potassium, mono-, di- ou tri- éthanolammonium) ; les teneurs en savons d'acides gras sont généralement de plus de 25% en poids de la formulation, généralement de 30 à 95% en poids.

Dans un pain de toilette dont la formulation repose sur d'autres constituants principaux que les savons d'acides gras, on trouve dans la formulation de 0 à 50% en poids, préférentiellement de 1 à 40% en poids de ces savons d'acides gras.

Ces compositions de pains de toilettes peuvent aussi contenir de 0 à 95%, de préférence de 0 à 60% de tensioactifs.

De 1 à 15% d'acides gras libres en $C_8$-$C_{22}$ peuvent aussi être introduits dans les compositions de savons en tant qu'agents surgraissants ou pour modifier l'aspect et le caractère crémeux de la mousse lors du lavage.

On peut aussi retrouver dans ces compositions des cires comme des cires paraffiniques, des cires naturelles comme la cire d'abeille ou l'ozokérite ou des cires silicones. Ces cires sont avantageusement utilisées pour améliorer l'aspect, la tenue, la processabilité et la conservation au stockage des pains de toilettes.

Les shampooings, et d'une manière plus générale les compositions détergentes pour l'usage personnel comme les gels douche, les gels nettoyants et les lotions, peuvent contenir, outre les tensio-actifs de l'invention, les additifs habituels présents dans ces types de formulation.

On peut citer en particulier :

- des agents tensio-actifs anioniques, tels que les sels hydrosolubles d'alkylsulfates, d'alkyléthersulfates, les alkyliséthionates et les alkyltaurates ou leurs sels, les alkylcarboxylates, les alkylsulfosuccinates ou les alkylsuccinamates, les alkylsarcosinates, les dérivés alkylés d'hydrolysats de protéines, les acylaspartates, les sels d'acides gras en $C_8$-$C_{18.}$
- des agents tensio-actifs non-ioniques, comme les dérivés polyoxyéthylénés d'alcools aliphatiques ou arylaliphatiques en $C_8$-$C_{22}$, les alkylpolysaccharides présentant un groupement hydrophobe en $C_6$-$C_{30}$, de préférence en $C_{10}$-$C_{16}$ et un groupement polysaccharide, par exemple polyglycoside, comme groupement hydrophile ainsi que de 1 à 3 unités sucre, les dérivés alkylés d'aminosucres, comme les alkylglucamides produits par la réaction d'amidification d'un acide gras sur la N-méthylglucamine
- des amides dérivés d'acides gras utilisés généralement pour leur aptitude à améliorer le moussage des compositions ou à augmenter la viscosité lesdites compositions ; ces amides sont choisis généralement
- des agents conditionneurs (améliorant la peignabilité, le coiffage, le toucher et le volume de la chevelure) cationiques tels que les polymères cationiques synthétiques du type MIRAPOL X® ou MIRAPOL A550® commercialisés par RHONE-POULENC, des polymères cationiques naturels comme les dérivés cationiques du guar (JAGUAR C13 S®, JAGUAR C162® commercialisés par RHONE-POULENC) ou les dérivés cationiques de la cellulose (POLYMER JR400® commercialisé par UNION CARBIDE).
- des organopolysiloxanes utilisés tels quels ou en solution dans un de leurs solvants usuels (huiles silicones de basses masses, huiles paraffiniques très ramifiées, les esters gras par exemple du type palmitate d'isopropyle, ...), comme agents conditionneurs ou de brillance.
- des agents antipelliculaires, comme les pyridinesthiones, plus spécialement la zinc pyridinethione, les composés à base de sélénium comme le sulfure de sélénium ou l'OCTOPYROX®, commercialisé par HOECHST
- des agents anti-parasites (anti-poux), comme le LINDANE ou différentes pyréthrines utilisées à ce propos.

Ces compositions peuvent aussi contenir des agents modifiant l'aspect ou la viscosité des formulations comme les composés perlescents à base de stéarate de polyéthylène glycol ou des polymères améliorant la viscosité ou la stabilité comme les CARBOPOL® commercialisés par GOODRICH, les hydrocolloïdes et leurs dérivés comme le guar ou les guars modifiés, la caroube, la gomme xanthane, les dérivés de la cellulose (hydroxyéthylcellulose, carboxyméthylcellulose).

Les compositions classiques de shampooings ou gels nettoyants contenant les tensio-actifs de l'invention) peuvent être constituées de (pourcentages en poids)

- tensio-actif de l'invention 1-15%
- agent tensio-actif anionique 0-15%
- agent tensio-actif non-ionique 0-15%
- alcanolamide d'acide gras 0-5%
- agent épaississant 0-5%
- agent conditionneur 0-3%
- parfum 0-2%
- conservateur 0-2%
- agent antipelliculaire 0-2%
- eau complément à 100%

Les tensio-actifs de type amphotère ou bétaïne de l'invention peuvent être utilisées également dans des formulations où il est nécessaire de maintenir en suspension dans l'eau des solides finement divisés, comme les formulations de matières actives agrochimiques (herbicides, insecticides, fongicides, ...) connues sous le nom générique de "suspensions concentrées".

Outre un tensio-actif dispersant, on retrouve comme additifs dans une formulation de suspension concentrée, des additifs comme ceux décrits dans la brochure commerciale "Auxiliaries for agrochemical formulations" éditées par RHÔNE-POULENC GERONAZZO SpA.

On peut citer par exemple un tensio-actif mouillant, pris parmi les dérivés alcoxylés d'alcools arylaliphatiques, les dérivés aryl sulfonés comme l'isopropylnaphtalène sulfonate de sodium, commercialisé sous le nom SUPRAGIL WP® par RHONE-POULENC GERONAZZO, les dialkyls sulfosuccinates comme le di-éthyl-2-hexyl sulfosuccinate de sodium, des polymères dispersants, comme les acides polyacryliques et leurs sels, les copolymères anhydride (ou acide) maléique - diisobutylène et leurs sels comme le GEROPON T36® (RHONE-POULENC GERONAZZO), les methylnaphtalène sulfonate de sodium condensés comme le SUPRAGIL MNS90® (RHONE-POULENC GERONAZZO), les polymères dispersants dérivés de la lignine comme les lignosulfonates de sodium ou de calcium ou d'autres tensioactifs dispersants comme les dérivés alcoxylés, éventuellement sulfatés ou phosphaté de tristyrylphénols. On peut trouver en outre dans ces formulations, des additifs antigels comme le propylèneglycol et des additifs épaississants, modifiant le comportement rhéologique de la suspension comme la gomme xanthane, les dérivés de la cellulose (carboxyméthylcellulose), la gomme guar ou ses dérivés, des argiles ou des argiles modifiées comme la bentonite et les bentones.

Parmi les matières actives pouvant être formulées ainsi, on trouve généralement celles dont le point de fusion est supérieur à 45 °C, préférentiellement supérieur à 60°C et dont la solubilité dans l'eau est inférieure à 10 g/l, préférentiellement inférieure à 1 g/l. Les matières actives phytosanitaires concernées sont les herbicides, les fongicides et insecticides, tels que ceux décrits dans THE PESTICIDE MANUAL (9° édition , C.R. WORKLING et R.J. HANCE , éditeurs ,publié par The British Crop Protection Council) et répondant aux critères ci-dessus.

La présente invention a également pour objet les compositions détergentes à base des tensio-actifs de type amphotère ou bétaïne faisant l'objet de l'invention, lesdites tensio-actifs de type amphotère ou bétaïne pouvant être présentes à raison d'environ 1 à 40% du poids desdites compositions.

Par composition détergente, on entend toute formulation lessivielle, poudre ou liquide, destinée à un emploi tant en machine à laver le linge, lave-vaisselle que pour les nettoyages ménagers en général en machine ou à la main comme les liquides pour lavage à la main de la vaisselle. Dans le domaine de la détergence ménagère, la présente invention vise plus particulièrement les compositions pour liquides de lavage de la vaisselle à la main.

Lesdites compositions contiennent de l'ordre de 0,1 à 20%, de préférence de l'ordre de 0,5 à 10% de leur poids de tensio- actifs purifiés selon l'invention et de l'ordre de 0,5 à 40%, de préférence de l'ordre de 1 à 20% de leur poids d'au moins un agent tensio-actif anionique ou non-ionique tel que décrit ci-après.

A côté desdits tensio-actifs de type amphotère ou bétaïne de l'invention, peuvent être présents dans les compositions détergentes d'autres additifs du type de ceux décrits ci-après.

☐ AGENTS TENSIO-ACTIFS autres, en quantités correspondant à environ 3-40% en poids par rapport à la composition détergente, agents tensio-actifs tels que
<u>agents tensio-actifs anioniques</u>

- les alkylesters sulfonates de formule R-CH($SO_3$M)-COOR', où R représente un radical alkyle en $C_{8}$-$_{20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium, ...) ou dérivé d'une alcanolamine (monoéthanolamine, diéthanolamine, triéthanolamine, ...). On peut citer tout particulièrement les méthyl ester sulfonates dont les radical R est en $C_{14}$-$C_{16}$ ;

- les alkylsulfates de formule $ROSO_3M$, où R représente un radical alkyle ou hydroxyalkyle en $C_{10}$-$C_{24}$, de préférence en $C_{12}$-$C_{20}$ et tout particulièrement en $C_{12}$-$C_{18}$, M représentant un atome d'hydrogène ou un cation de même définition que ci-dessus, ainsi que leurs dérivés éthoxylénés (OE) et/ou propoxylénés (OP), présentant en moyenne de 0,5 à 6 motifs, de préférence de 0,5 à 3 motifs OE et/ou OP ;
- les alkylamides sulfates de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
- les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB-A-1 082 179, les sulfonates de paraffine, les N-acyl N-alkyltaurates, les alkylphosphates, les iséthionates, les alkylsuccinamates les alkylsulfosuccinates, les monoesters ou diesters de sulfosuccinates, les N-acyl sarcosinates, les sulfates d'alkylglycosides, les polyéthoxycarboxylates le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;

<u>agents tensio-actifs non-ioniques</u>

- les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy ;
- les glucosamide, glucamide, glycérolamide ;
- les alcools aliphatiques en $C_8$-$C_{22}$ polyoxyalkylénés contenant de 1 à 25 motifs oxyalkylènes (oxyéthylène, oxypropylène); à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.
- les produits résultant de la condensation de l'oxyde d'éthylène le composé résultant de la condensation de l'oxyde de propylène avec le propylène glycol, tels les PLURONIC commercialisés par BASF ;
- les produits résultant de la condensation de l'oxyde d'éthylène le composé résultant de la condensation de l'oxyde de propylène avec l'éthylènediamine, tels les TETRONIC commercialisés par BASF ;
- les oxydes d'amines tels que les oxydes d'alkyl $C_{10}$-$C_{18}$ diméthylamines, les oxydes d'alkoxy $C_8$-$C_{22}$ éthyl dihydroxy éthylamines;
- les alkylpolyglycosides décrits dans US-A-4 565 647 ;
- les amides d'acides gras en $C_8$-$C_{20}$
- les acides gras éthoxylés
- les amides gras éthoxylés
- les amines éthoxylées

<u>agents tensio-actifs cationiques</u>

- les halogénures d'alkyldiméthylammonium

☐ AGENTS "BUILDERS" (adjuvants améliorant la performance des agents de surface), en quantités correspondant à environ 5-50%, de préférence à environ 5-30% en poids pour les formules détergentes liquides, ou à environ 10-80%, de préférence 15-50% en poids pour les formules détergentes en poudres, agents builders tels que <u>"builders" inorganiques</u>

- les polyphosphates (tripolyphosphates, pyrophosphates, orthophosphates, hexamétaphosphates) de métaux alcalins, d'ammonium ou d'alcanolamines
- les tétraborates ou les précurseurs de borates
- les silicates, en particulier ceux présentant un rapport $SiO_2/Na_2O$ de l'ordre de 1,6/1 à 3,2/1 et les silicates lamellaires décrits dans US-A-4 664 839
- les carbonates (bicarbonates, sesquicarbonates) alcalins ou alcalino-terreux
- les cogranulés de silicates hydratés de métaux alcalins et de carbonates de métaux alcalins (sodium ou de

potassium) riches en atomes de silicium sous forme Q2 ou Q3, décrits dans EP-A-488 868

- les aminosilicates cristallins ou amorphes de métaux alcalins (sodium, potassium) ou d'ammonium, tels que les zéolithes A, P, X, ...; la zélote A de taille de particules de l'ordre de 0,1-10 micromètres est préférée

"builders" organiques

- les polyphosphonates hydrosolubles (éthane 1-hydroxy-1, 1-diphosphonates, sels de méthylène diphosphonates, ...)
- les sels hydrosolubles de polymères ou de copolymères carboxyliques ou leurs sels hydrosolubles tels que
    - les éthers polycarboxylates (acide oxydisuccinique et ses sels, tartrate monosuccinic acide et ses sels, tartrate disuccinic acide et ses sels
    - les éthers hydroxypolycarboxylates
    - l'acide citrique et ses sels, l'acide mellitique, l'acide succinique et leurs sels
    - les sels d'acides polyacétiques (éthylènediaminetetraacétates, nitrilotriacétates N-(2 hydroxyéthyl)-nitrilodiacétates)
    - les acides alkyl C5-C20 succiniques et leurs sels( 2-dodécénylsuccinates, lauryl succinates, ...)
    - les esters polyacétals carboxyliques
    - l'acide polyaspartique, l'acide polyglutamique et leurs sels
- les polyimides dérivés de la polycondensation de l'acide aspartique et/ou de l'acide glutamique
- les dérivés polycarboxyméthylés de l'acide glutamique ou d'autres acides aminés.

☐ AGENTS DE BLANCHIMENT, en quantités d'environ 0,1-20%, de préférence environ 1-10% en poids, éventuellement associés à des ACTIVATEURS DE BLANCHIMENT, en quantités d'environ 0,1-60%, de préférence d'environ 0,5-40% en poids, agents et activateurs tels que
agents de blanchiment

- les perborates tels que le perborate de sodium monohydraté ou tétrahydraté
- les composés peroxygénés tels que le carbonate de sodium peroxyhydraté, le pyrophosphate peroxyhydraté, l'urée peroxyhydratée, le peroxyde de sodium, le persulfate de sodium

de préférence associés à un activateur de blanchiment générant in situ dans le milieu lessiviel, un peroxyacide carboxylique ; parmi ces activateurs, on peut mentionner, la tetraacétyléthylène diamine, la tetraacétyl méthylène diamine, le tetraacétyl glycoluryl, le p-acétoxybenzène sulfonate de sodium, le pentaacétyl glucose, l'octaacétyl lactose, ...

- les acides percarboxyliques et leurs sels (appelés "percarbonates") tels que le monoperoxyphtalate de magnésium hexahydraté, le métachloroperbenzoate de magnésium, l'acide 4-nonylamino-4-oxoperoxybutyrique, l'acide 6-nonylamino-6-oxoperoxycaproique, l'acide diperoxydodécanedioique, le nonylamide de l'acide peroxysuccinique, l'acide décyldiperoxysuccinique.

Ces agents peuvent être associés à au moins un des agents anti-salissures ou anti-redéposition mentionnés ci-après.

Peuvent également être mentionnés des agents de blanchiments non oxygénés, agissant par photoactivation en présence d'oxygène, agents tels que les phtalocyanines d'aluminium et/ou de zinc sulfonées

☐ AGENTS ANTI-SALISSURES, en quantités de l'ordre de 0,01-10%, de préférence environ 0,1-5%, et tout particulièrement de l'ordre de 0,2-3% en poids, agents tels que

- les dérivés cellulosiques tels que les hydroxyéthers de cellulose, la méthylcellulose, l'éthylcellulose, l'hydroxypropyl méthylcellulose, l'hydroxybutyl méthylcellulose
- les polyvinylesters greffés sur des troncs polyalkylènes tels que les polyvinylacétates greffés sur des troncs polyoxyéthylènes (EP-A-219 048)
- les alcools polyvinyliques
- les copolymères polyesters à base de motifs éthylène téréphtalate et/ou propylène téréphtalate et polyoxyéthylène téréphtalate, avec un rapport molaire (nombre de motifs) ethylène téréphtalate et/ou propylène téréphtalate / (nombre de motifs) polyoxyéthylène téréphtalate de l'ordre de 1/10 à 10/1, de préférence de l'ordre

de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666) ;

- les oligomères polyesters sulfonés obtenus par sulfonation d'un oligomère dérivé de l'alcool allylique éthoxylé, du diméthyltéréphtalate et du 1,2 propylène diol, présentant de 1 à 4 groupes sulfonés (US-A-4 968 451)
- les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
- les polyesters-polyuréthanes obtenus par réaction d'un polyesters de masse moléculaire en nombre de 300-4000 obtenus à partir d'acide adipique et/ou d'acide téréphtalique et/ou d'acide sulfoisophtalique et d'un diol de masse inférieure à 300, sur un prépolymère à groupements isocyanates terminaux obtenus à partir d'un polyoxyéthylène glycol de masse moléculaire de 600-4000 et d'un diisocyanate ((FR-A-2 334 698)

☐ AGENTS ANTI-REDEPOSITION, en quantités d'environ 0,01-10% en poids pour une composition détergente en poudre, d'environ 0,01-5% en poids pour une composition détergente liquide, agents tels que

- les monoamines ou polyamines éthoxylées, les polymères d'amines éthoxylées (US-A-4 597 898, EP-A-1 1 984)
- la carboxyméthylcellulose
- les oligomères polyesters sulfonés obtenus par condensation de l'acide isophtalique, du sulfosuccinate de diméthyle et de diéthylène glycol (FR-A-2 236 926)
- les polyvinylpyrollidones

☐ AGENTS CHELATANTS du fer et du magnésium, en quantités de l'ordre de 0,1-10%, de préférence de l'ordre de 0,1-3% en poids, agents tels que

- les aminocarboxylates tels que les éthylènediaminetétraacétates, hydroxyéthyl éthylènediaminetriacétates, nitrilotriacétates
- les aminophosphonates tels que les nitrilotris(méthylène phosphonates)
- les composés aromatiques polyfonctionnels tels que les dihydroxydisulfobenzènes

☐ AGENTS DISPERSANTS POLYMERIQUES, en quantité de l'ordre de 0,1-7%, en poids pour contrôler la dureté en calcium et magnésium, agents tels que

- les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acrylique, acide ou anhydride maléique, acide fumarique, acide itaconique, acide aconitique, acide mésaconique, acide citraconique, acide méthylènemalonique, et tout particulièrement les polyacrylates de masse moléculaire de l'ordre de 2 000 à 10 000 (US-A-3 308 067), les copolymères d'acide arylique et d'anhydride maléique de masse moléculaire de l'ordre de 5 000 à 75 000 (EP-A-66 915)
- les polyéthylèneglycols de masse moléculaire de l'ordre de 1000 à 50 000

☐ AGENTS DE FLUORESCENCE (BRIGHTENERS), en quantité d'environ 0,05-1,2% en poids, agents tels que les dérivés de stilbène, pyrazoline, coumarine, acide fumarique, acide cinnamique, azoles, méthinecyanines, thiophènes, ... ("The production and application of fluorescent brightening agents" - M. Zahradnik, publié par John Wiley & Sons, New York-1982-)

☐ AGENTS SUPPRESSEURS DE MOUSSES, en quantités pouvant aller jusqu'à 5% en poids, agents tels que

- les acides gras monocarboxyliques en $C_{10}$-$C_{24}$ ou leurs sels alcalins, d'ammonium ou alcanolamines, les triglycérides d'acides gras
- les hydrocarbures saturés ou insaturés aliphatiques, alicycliques, aromatiques ou hétérocycliques, tels que les paraffines, les cires
- les N-alkylaminotriazines
- les monostéarylphosphates, les monostéaryl alcool phosphates
- les huiles ou résines polyorganosiloxanes éventuellement combinées avec des particules de silice

☐ AGENTS ADOUCISSANTS, en quantités d'environ 0,5-10% en poids, agents tels que les argiles

☐ ENZYMES en quantité pouvant aller jusqu'à 5 mg en poids, de préférence de l'ordre de 0,05-3mg d'enzyme active /g de composition détergente, enzymes telles que
les protéases, amylases, lipases, cellulases, peroxydases (US-A-3 553 139, US-A-4 101 457, US-A-4 507 219, US-A-4 261 868

☐ AUTRES ADDITIFS tels que

- des alcools (méthanol, éthanol, propanol, isopropanol, propanediol, éthylène glycol, glycérine)
- des agents tampons
- des parfums
- des pigments

Les tensio-actifs de type amphotère ou bétaïne de l'invention peuvent en outre être utilisés comme agent de surface des textiles (lubrification, igniifugation, adoucissage ...), comme agent de surface des métaux (anti-corrosion, lubrification ...) et comme agent antistatique pour fibres et films de polymères minéraux ou organiques. Ils servent également au nettoyage des lentilles optiques ou d'accessoires rentrant en contact prolongé avec les muqueuses ou les yeux.

Les exemples suivants illustrent l'invention sans en limiter la portée.

EXEMPLE 1

On utilise comme électrodialyseur, un électrodialyseur pilote de type TS-2-10P commercialisé par la Société Eurodia. Cet électrodialyseur comporte une surface membranaire totale utile de 2000 $cm^2$ et comprend 10 cellules à 2 compartiments de 200 $cm^2$ de surface unitaire utile.

Les membranes anioniques et cationiques utilisées sont des membranes commercialisées par la Société Eurodia ou Tokoyama Soda dont les références sont respectivement Neosepta AM1 et Neosepta CMX.

On électrodialyse 2 litres de solution de cocoamphomonoacètatel. Ce produit de départ a été préparé par réaction de cocoimidazoline sur l'acide chloroacétique conformément à l'exemple 3 de la demande de brevet européen EP-94.402.226 dont le pH est réglé à 7 par addition d'HCl et additionnée de 9% en poids par rapport au poids du tensio-actif de propylène glycol.

On opère à température ambiante avec une densité de courant de 18,4 $mA/cm^2$. La teneur initiale en NaCl est de 8,5% par rapport au poids total de la solution. La viscosité finale de la solution est de 44 mPa.s.

On arrête l'électrodialyse au bout de 8h.

On dose le NaCl à la fin et on trouve une valeur de 0,6%, le glycolate de Na est également dosé et on constate qu'il passe d'une teneur de 1,2% à 0,6%. La viscosité finale est de 71 mPa.s. La concentration en produit de formule (5) passe de 26% à 33%.

EXEMPLE COMPARATIF 2

On répète exactement le mode opératoire de l'exemple 1, sauf que la solution de tensio-actif ne comporte pas de polyol et a une viscosité initiale de 200 mPa.s. Au bout de 2h d'électrodialyse, la teneur en NaCl finale est de 2,5% et la teneur en glycolate est restée à 1,2%. La viscosité finale est supérieure à 400 mPa.s. On constate en outre une baisse de l'efficacité de l'électrodialyse provenant du colmatage des membranes anioniques. Ce colmatage a pour conséquence une baisse du rendement faradique et un allongement drastique du temps d'électrodialyse avant d'atteindre un taux de dessalement satisfaisant.

EXEMPLE 3

On répète exactement le mode opératoire de l'exemple 1, sauf que l'imidazoline de départ est préparée par réaction d'huile de coprah sur l'aminoéthanolamine. La solution de tensio-actif avant électrodialyse comporte 6% en poids de glycérol uniquement engendré par la réaction de départ.

La teneur en NaCl passe de 7,9% à 0,1% et la teneur en glycolate passe de 1,7% à 0,1%.

EXEMPLE 4 - Préparation d'une composition de dentifrice gel

On prépare de façon séparée par simple mélange les 4 phases A, B, C et D ci-dessous.

Phase A:

- ☐ Eau 20,65%
- ☐ Caraghenanne 1
- ☐ NaF 0,23
- ☐ Benzoate de Na 0,5
- ☐ Saccharinate de Na 0,020

Phase B:

- ☐ Sorbitol 30,00
- ☐ Glycérine 20,00
- ☐ Maltilol 5,00

Phase C :

- ☐ Silice TIXOSIL 73 17%

Phase D :

- ☐ Arôme 1%
- ☐ Tensio-actif issu de l'exemple 1 4,5%
- ☐ Colorant 0,1 %

On disperse sous forte agitation les composants de la phase A à 50°C. On ajoute ensuite la phase B puis la silice. L'introduction de la phase D se fait sous agitation modérée et sous vide pour éviter la formation de bulles. On obtient ainsi une pâte dentaire, bien tolérée par les muqueuses.

Le produit issu de l'exemple 1 est préférable aux produits commerciaux contenant de forte quantité de sel car il ne communique pas de goût salé ou amer à la pâte dentaire. Il contient moins d'impuretés et est donc plus adapté au contact avec les muqueuses.

<u>EXEMPLE 5 - Préparation d'une composition de Bain-Bouche</u>

On prépare de façon séparée par simple mélange les 3 phases A, B, et C ci-dessous.

Phase A:

- ☐ Eau 74,785
- ☐ Allantoïne 0,050
- ☐ Glycérine 10,00
- ☐ Maltilol 5,00
- ☐ NaF 0,050
- ☐ Extrait de Mauve 0,100

Phase B :

- ☐ Polysorbate 60 O,6
- ☐ Arôme 0,05
- ☐ Alcool 8,00

Phase C :

- ☐ Tensio-actif de l'exemple 1 1%
- ☐ saccharinate de sodium 0,015%
- ☐ Benzoate de sodium 0,3%
- ☐ acide lactique pour pH 6,4

La composition de bain-bouche est obtenue par simple mélange des phases A, B et C.

EXEMPLE 6 - Préparation d'une composition de Bain-Bouche

| | |
|---|---|
| Alcool | 6% |
| Glycérol | 2% |
| Benzoate de Na | 2% |
| Bicarbonate de Na | 1,5% |
| Saccharinate de Na | 0,2% |
| Allantoïne | 0,3% |
| Gomme Xanthane (RHODICARE X 401) | 0,2% |
| Tensio-actif de l'exemple 1 | 2,5% |
| Arôme menthe hydrosoluble | 2% |
| Polysorbate 20 | 1 % |
| Colorant | qs |
| Eau distillée | qs 100 |
| Acide citrique | qs pour pH 4,7 |

EXEMPLE 7 - Préparation d'un dentifrice liquide 2 en 1

De composition suivante :

| | |
|---|---|
| Gomme Xanthane (RHODICARE X 401) | 0,3% |
| Sorbitol (70%) | 69,4% |
| Monofluorophosphate de Na | 0,76% |
| Saccharinate de Na | 0,2% |
| Benzoate de Na | 0,2% |
| Eau | qs p100 |
| Silice TIXOSIL 73 | 15,0% |
| Colorant | qs |
| Produit de l'exemple 1 | 4,2% |
| Arôme | qs |

On solubilise les sels de sodium dans l'eau et on ajoute sous agitation de sorbitol. On hydrate la gomme xanthane dans ce mélange sous forte agitation. On laisse reposer 30 mn. on ajoute la silice et le colorant sous agitation pendant 15 mn. On dégage la solution sous vide pendant 45 mn. On ajoute l'arôme et le tensio-actif sous vide et agitation modérée.

**Revendications**

1. Procédé de purification de tensio-actif de type bétaïne ou amphotère selon lequel on soumet une solution aqueuse dudit tensio-actif à une électrodialyse, caractérisé en ce que ladite solution contient de 0,1 à 20%, de préférence de 0,5 à 10% en poids d'un alcool, par rapport au poids du tensio-actif.

2. Procédé selon la revendication 1, caractérisé en ce que l'alcool est un polyol.

3. Procédé selon la revendication 1 ou 2, caractérisé en ce que le tensio-actif de type bétaïne est choisi parmi :

   - ☐ les bétaïnes de formule 1 :

$$R_1\ R_2\ R_3\ N\ R_4^+\ C(O)O^- \qquad (1)$$

   - ☐ Les sulfo-bétaïnes de formule 2 :

$$R_1\ R_2\ R_3\ N\ R_4^+\ SO_3^- \qquad (2)$$

□ les amidoalkylbétaïnes de formule 3 :

$$R_1 C(O) - NH\, R_2^+\, N\, (R_3\, R_4)\, R_5\, C(O)O^- \qquad (3)$$

□ et les sulfo-bétaïnes de formule 4 :

$$R_1 C(O) - NH\, R_2 N^+\, (R_3\, R_4)\, R_5\, SO_3^- \qquad (4)$$

formules dans lesquelles les radicaux $R_1$ représente un radical alkyle ou alcényle de 10 à 24 atomes de carbone, $R_2$, $R_3$, $R_4$, et $R_5$ identiques ou différents représentent un radical alkyle ou alkylène ayant de 1 à 4 atomes de carbone.

**4.** Procédé selon la revendication 3, caractérisé en ce que le tensio-actif est un amphoacétate de formule :

$$R_1\, C(O) - NH - CH_2CH_2 - \underset{\displaystyle CH_2\, C(O)O\, Na}{\underset{|}{N}} - CH_2CH_2 - OH \qquad (5)$$

dans laquelle $R_1$ a la signification donnée à la revendication 3.

**5.** Procédé selon l'une quelconque des revendications précédentes, caractérisé en ce que l'alcool comporte par molécule, au moins 2 groupes hydroxy dont la partie hydrocarbonée comporte de 2 à 20 atomes de carbone.

**6.** Procédé selon la revendication 5, caractérisé en ce que l'alcool est choisi parmi le dipropylèneglycol, le propanediol 1-2, le butanediol 1-3 et le glycérol.

**7.** Procédé selon l'une quelconque des revendications précédentes, caractérisé en ce qu'on règle le pH de la solution aqueuse de tensio-actif entre 6 et 8 durant l'électrodialyse.

**8.** Procédé de la revendication 7, caractérisé en ce que le pH est de 7.

**9.** Procédé selon l'une quelconque des revendications 4 à 8, caractérisé en ce que tensio-actif électrodialysé provient de la réaction d'une alkylimidazoline de formule (6).

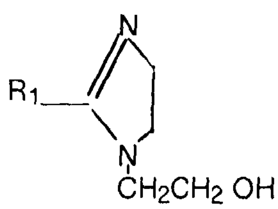

(6)

dans laquelle $R_1$ a la signification donnée à la revendication ou l'un de ses dérivés à cycle ouvert avec un acide monohalogénoacétique ou l'un de ses sels en présence d'un alcali.

**10.** Procédé selon la revendication 9, caractérisé en ce que l'imidazoline de formule (6) est préparée par réaction d'un triglycéride gras sur l'aminoéthyle éthanolamine.

**11.** Procédé selon la revendication 10, caractérisé en ce que le pH est réglé de manière à ce qu'il ne dépasse pas 10.

**12.** Procédé selon l'une quelconque des revendications 4 à 9, caractérisé en ce qu'on fait réagir une aminoalkylamine de formule 7 :

$$R_1\ C(O)\ NH\ R_2\ NH\ R_3 - OH \qquad (7)$$

dans laquelle $R_1$, $R_2$ et $R_3$ ont la signification indiquée à la revendication 3 avec du formaldéhyde et un cyanure de formule (8) :

$$XCN \qquad (8)$$

dans laquelle X représente un atome d'hydrogène ou un métal alcalin, sous réserve que si X est un atome d'hydrogène, on hydrolyse le nitrure obtenu avec l'alcali.

**13.** Tensio-actif obtenu par la mise en oeuvre d'un procédé selon l'une quelconque des revendications 1 à 12.

**14.** Compositions cosmétiques ou détergentes contenant un tensio-actif tel que défini à la revendication 13.

**15.** Compositions selon la revendication 14, caractérisées en ce qu'elles sont un dentifrice ou un bain de bouche.

**16.** Compositions selon la revendication 14, caractérisées en ce qu'elles sont des shampooings, des compositions moussantes ou nettoyantes de la peau ou des muqueuses.

**17.** Compositions selon la revendication 14, caractérisées en ce qu'elles servent au nettoyage des lentilles optiques ou d'accessoires rentrant en contact prolongé avec les muqueuses ou les yeux.

Office européen des brevets

# RAPPORT DE RECHERCHE EUROPEENNE

Numero de la demande
EP 96 40 0728

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | FR-A-2 283 950 (ALBRIGHT &WILSON) 2 Avril 1976<br>* page 1, ligne 38 - page 2, ligne 9 *<br>* page 2, ligne 28 - page 4, ligne 20 *<br>* page 4, ligne 32 - ligne 39 *<br>* page 6, ligne 13 - ligne 33 * | 1,3,4,7, 8 | C07C227/40<br>C07C231/24<br>C07C303/44<br>C07C229/12<br>C07C233/36<br>C07C309/14<br>B01D61/44<br>C11D1/88<br>C11D1/90<br>C11D1/92<br>C11D3/00 |
| X | * exemples * | 13 | |
| A | CHEMICAL ABSTRACTS, vol. 115, no. 21, 25 Novembre 1991<br>Columbus, Ohio, US;<br>abstract no. 231682,<br>KOJIMA, KUNIHIKO ET AL.: "Manufacture of high-purity quaternary ammonium hydroxide by electrodialysis"<br>page 891;<br>XP002008128<br>* abrégé *<br>& JP-A-03 093 752 (TOYO GASEI KOGYO) | 2,5,6 | |
| A,D | EP-A-0 269 939 (KAO CORPORATION)<br>* page 26, ligne 4 - page 29 * | 1,4,9 | DOMAINES TECHNIQUES RECHERCHES (Int.Cl.6) |
| X | * page 35, dernier alinéa; revendication 1 * | 13,14,16 | C07C<br>C11D<br>B01D |
| A | DATABASE WPI<br>Week 9247<br>Derwent Publications Ltd., London, GB;<br>AN 92-386074<br>XP002008129<br>& JP-A-04 284 832 (ASAHI CHEM. IND. CO.)<br>* abrégé * | 1,4 | |

Le présent rapport a été établi pour toutes les revendications

| Lieu de la recherche | Date d'achèvement de la recherche | Examinateur |
|---|---|---|
| LA HAYE | 11 Juillet 1996 | Seufert, G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)